# EUROPEAN PATENT APPLICATION

(11) **EP 4 086 619 A1**
(43) Date of publication of application: **09.11.2022**
(21) Application number: 21764588.6
(22) Date of filing: 03.02.2021
(51) Int. Cl.: G01N 27/68, G01N 27/70

(54) **GAS MEASUREMENT DEVICE AND GAS MEASUREMENT METHOD**

(30) Priority: 05.03.2020 JP 2020038060
(71) Applicant: Sintokogio, Ltd., Nagoya-shi, Aichi 450-6424 (JP); National University Corporation TOYOHASHI UNIVERSITY OF TECHNOLOGY, Toyohashi-shi, Aichi 441-8580 (JP)
(72) Inventor: SUZUKI, Yoshihisa, Toyokawa-shi, Aichi 442-8505 (JP); NODA, Toshihiko, Toyohashi-shi, Aichi 441-8580 (JP); SAWADA, Kazuaki, Toyohashi-shi, Aichi 441-8580 (JP)
(74) Representative: Hoffmann Eitle
(86) International application number: PCT/JP2021/003961
(87) International publication number: WO 2021/176937

(57) **Abstract**

The gas measurement device includes a plurality of electrodes arranged apart from each other so as to have a gap through which gas can pass, each electrode connected to a power source for applying voltage or current, and a gas sensor configured to detect gas molecules passing between the electrodes.

## Description

### Technical Field

The present disclosure relates to a gas measurement device and a gas measurement method.

### Background Art

Patent Document 1 discloses a gas sensor. The gas sensor includes a reaction film that chemically reacts with a specific gas, and a pair of electrodes connected to the reaction film. The gas sensor detects a resistance value between electrodes that changes in accordance with the composition of gas passing through the reaction membrane.

### Citation List

### Patent Document

Patent Document 1: Japanese Unexamined Patent Publication No. H04-216452

### Summary of Invention

### Technical Problem

In the gas sensor described in Patent Document 1, when a plurality of types of gases are to be measured, a plurality of types of reaction films are required to specify the types of gases. The present disclosure provides a gas measurement device and a gas measurement method capable of detecting a plurality of types of gases with a simple configuration.

### Solution to Problem

A gas measurement device according to one aspect of the present disclosure includes a plurality of electrodes and a gas sensor. The plurality of electrodes is arranged apart from each other so as to have a gap through which gas can pass, and each electrode is connected to a power source for applying voltage or current. The gas sensor detects gas molecules passing between the electrodes.

In the gas measurement device, a plurality of electrodes is provided, and electrostatic force is generated between the electrodes by applying voltage or current to the plurality of electrodes. The gas passes between the electrodes. At this time, the traveling direction of the ionized gas molecules contained in the gas changes due to the electrostatic force. The traveling direction of the non-ionized gas molecules does not change. The gas measurement device can change the type of gas molecules that can reach the gas sensor by changing magnitude of the electrostatic force. Therefore, the gas measurement device can detect a plurality of types of gases with a simple configuration as compared to a gas measurement device including a plurality of types of reaction films.

In one embodiment, the gas measurement device may further include a plurality of gas sensors including the gas sensor. The plurality of gas sensors is arranged along an arrangement direction of the plurality of electrodes. The traveling direction of the ionized gas molecules changes to the direction of either the anode or cathode of the electrode. For this reason, the gas measurement device can detect a plurality of types of gas which traveling direction has changed.

In one embodiment, the plurality of gas sensors may include another gas sensor capable of detecting gas molecules of a gas species different from the gas species of the gas molecules detected by the gas sensor. In this case, the gas measurement device can improve gas detection accuracy as compared with a gas measurement device including a gas sensor capable of detecting a single type of gas.

In one embodiment, a first region where the gas passing straight between the electrodes reaches and a first region adjacent to a second region may be set, and the gas sensor may be disposed in the second region. In this case, the gas measurement device can mainly measure ionized gas molecules.

In one embodiment, the gas measurement device may further include a signal generation unit, a control unit, an acquirement unit, and an output unit. The signal generation unit outputs a synchronous signal for determining timing. The control unit controls the power supply so that the voltage or current with magnitude determined by the control signal is output at the timing determined by the synchronous signal based on the control signal for determining the magnitude of the voltage or current and the synchronous signal. The acquirement unit acquires a detection value of the gas sensor at a timing determined by the synchronous signal. The output unit outputs the detection value and the control signal in association with each other. In this case, the gas measurement device can output the detection value and the synchronous signal in association with each other.

In one embodiment, the gas measurement device may further include a determination unit. The determination unit determines a gas species based on a pre-acquired relationship, the detection value and the control signal output by the output unit, wherein the pre-acquired relationship is between the gas species, the detection value, and the control signal. In this case, the gas measurement device can determine the gas species of the mixed gas including gas molecules having different gas traveling directions.

A gas measurement method according to another aspect of the present disclosure includes the following steps (1) to (4).
(1) applying voltage or current to a plurality of electrodes spaced apart from one another so as to have a gap through which gas can pass. Applying voltage or current is based on the control signal that determines magnitude of the voltage or current and the synchronous signal that determines the timing. The magnitude of the voltage or current is determined by a control signal. The timing is determined by a synchronous signal.
(2) detecting gas molecules passing between the electrodes by a gas sensor.
(3) acquiring a detection value detected by the gas sensor at a timing determined by the synchronous signal.
(4) determining a gas species based on a pre-acquired relationship, the acquired detection value and the control signal, wherein the pre-acquired relationship is relationship between the gas species, the control signal, and the detection value.

### Advantageous Effects of Invention

According to the gas measurement device and the gas measurement method of the present disclosure, a plurality of types of gases can be detected with a simple configuration.

### Brief Description of the Drawings

[FIG. 1] FIG. 1 is a cross-sectional view illustrating an example of a gas measurement device according to an embodiment.
[FIG. 2] FIG. 2 is a schematic view illustrating an example of the traveling direction of gas molecules with the space between the electrodes in FIG. 1 enlarged.
[FIG. 3] (A) of FIG. 3 is a cross-sectional view illustrating an example of a plurality of gas sensors arranged along an arrangement direction of a plurality of electrodes. (B) of FIG. 3 is a cross-sectional view illustrating an example of the gas sensor arranged in the second region.
[FIG. 4] (A) of FIG. 4 is a block diagram illustrating an example of the gas measurement device according to the embodiment. (B) of FIG. 4 is an example of the control signal and the synchronous signal.
[FIG. 5] FIG. 5 is a flowchart illustrating an example of a gas measurement method according to an embodiment.
[FIG. 6] FIG. 6 is a schematic view illustrating an example of a change in the traveling direction of gas molecules according to the strength of the electrostatic force of the electrode.

### Description of Embodiments

An embodiment of the present disclosure is described below with reference to the drawings. In the description below, the same or equivalent elements are denoted by the same reference characters, and overlapping description is not repeated. Dimension ratios of the drawings do not necessarily match with those described. Terms "up", "down", "left", and "right" are based on the illustrated states and are for convenience.

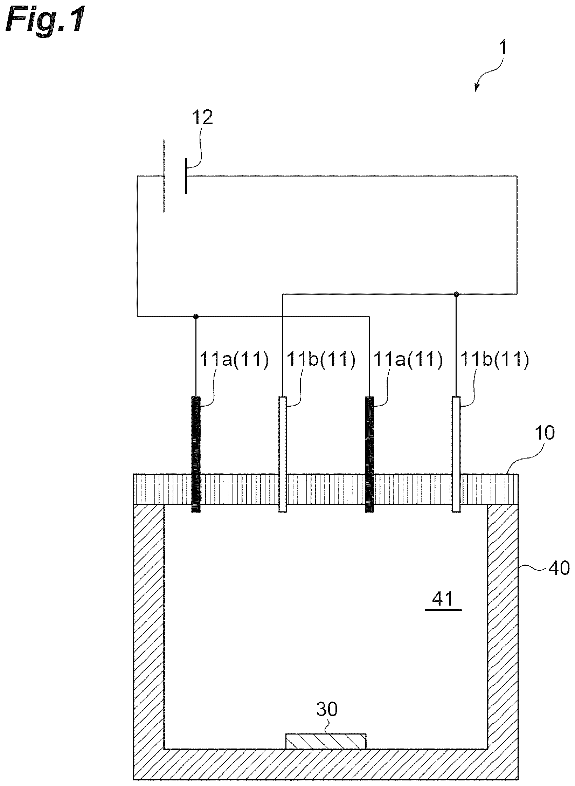

[Configuration of Gas Measurement Device] FIG. 1 is a cross-sectional view illustrating an example of a gas measurement device 1 according to an embodiment. The gas measurement device 1 shown in FIG. 1 is a device for measuring gas components. The gas measurement device 1 may be provided as an electric circuit component. As an example, the gas measurement device 1 is a micro electro mechanical systems (MEMS) device. The gas measurement device 1 includes a filter 10, a plurality of electrodes 11, a base member 40, and a gas sensor 30.

The base member 40 defines a space therein. The base member 40 is formed of a gas-impermeable material. The base member 40 is open at its upper portion and has an opening communicating with the space. The filter 10 is disposed so as to close the opening in the upper portion of the base member 40. The filter 10 is a substantially plate-shaped member and is formed of a material that allows gas to pass therethrough. The filter 10 and the base member 40 are joined so that there is no gap through which gas passes. Thus, the filter 10 and the base member 40 define a gas chamber 41.

The filter 10 is provided with a plurality of electrodes 11. Each of the plurality of electrodes 11 has a substantially plate shape and is arranged apart from each other. As an example, the plurality of electrodes 11 are provided in a direction perpendicular to the in-plane direction of the filter 10. The plurality of electrodes 11 are arranged in parallel along the in-plane direction of the filter 10. The plurality of electrodes 11 are each connected to a power source 12 capable of applying voltage or current. The plurality of electrodes 11 are connected to a positive electrode or a negative electrode of the power source 12, and constitute at least a pair of an anode and a cathode. A gap through which gas can pass is defined between each of the plurality of electrodes 11.

The gas sensor 30 is provided in the gas chamber 41. As an example, the gas sensor 30 is provided downstream of the plurality of electrodes 11, that is, on the passing side where the gas has passed between the electrodes 11. The gas sensor 30 detects gas molecules that pass between the electrodes 11 and adhere to a predetermined surface of the gas sensor 30.

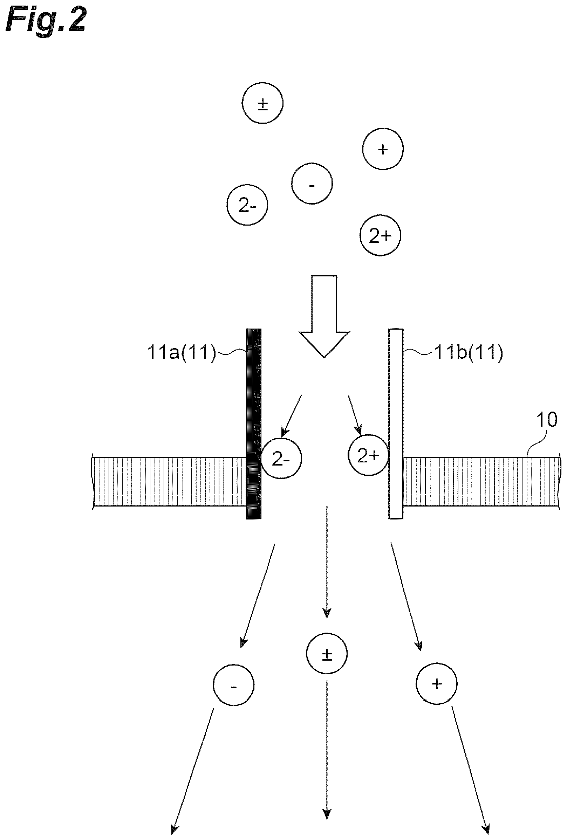

[Details of Electrodes Included in Filter] FIG. 2 is a schematic view showing the traveling direction of gas molecules with the space between the electrodes 11 provided in the filter 10 of FIG. 1 enlarged. In FIG. 2, as an example, the first electrode 11a is connected to the positive electrode of the power source 12 to form an anode. The second electrode 11b is connected to the negative electrode of the power source 12 to form a negative electrode.

The gas may include gas molecules that are not ionized, gas molecules that are ionized into anions, and gas molecules that are ionized into cations. The traveling directions of the ionized gas molecules contained in the gas passing between the first electrode 11a and the second electrode 11b is changed by the first electrode 11a and the second electrode 11b. The non-ionized gas molecules are not affected by the first electrode 11a and the second electrode 11b. The non-ionized gas molecules pass straight between the first electrode 11a and the second electrode 11b.

To be specific, attractive force due to electrostatic force is generated between the first electrode 11a which is positively charged and the negative ions which are negatively charged. Repulsive force due to the electrostatic force is generated between the negatively charged second electrode 11b and the negatively charged anions. The electrostatic force applied to the negative ions increases in accordance with the valence of the negative ions. As an example, the traveling direction of the anion (monovalent) passing between the first electrode 11a and the second electrode 11b changes to a direction approaching the first electrode 11a. The negative ions (divalent) passing between the first electrode 11a and the second electrode 11b greatly change their traveling direction toward the first electrode 11a, and are adsorbed by the first electrode 11a.

Similarly, the repulsive force is generated between the positively charged first electrode 11a and the positively charged positive ions. The attractive force is generated between the negatively charged second electrode 11b and the positively charged positive ions. The electrostatic force applied to the positive ions increases in accordance with the valence of the positive ions. As an example, the traveling direction of positive ions (monovalent) passing between the first electrode 11a and the second electrode 11b changes to a direction approaching the second electrode 11b. The positive ions (divalent) passing between the first electrode 11a and the second electrode 11b greatly change their traveling direction toward the second electrode 11b and are adsorbed by the second electrode 11b.

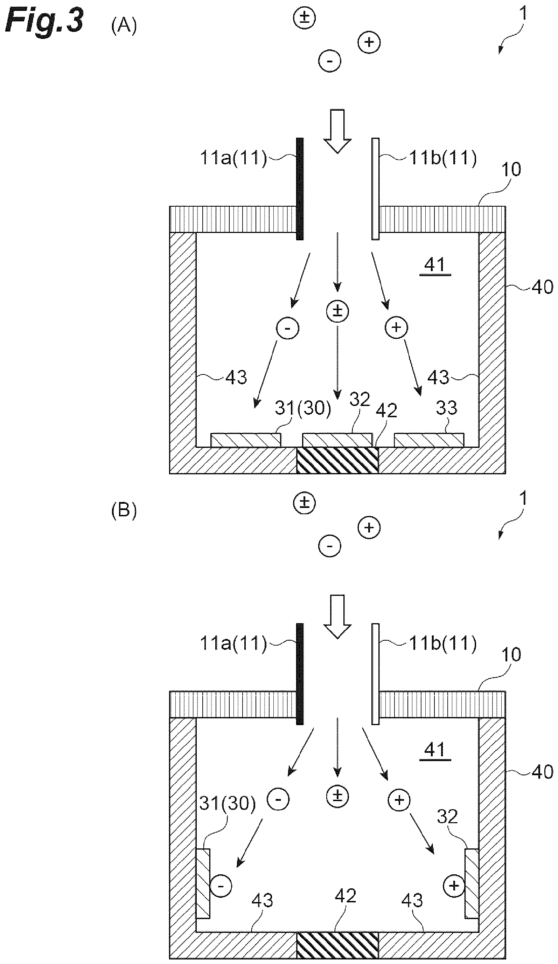

[Details of Configuration of Gas Sensor] (A) of FIG. 3 a is a cross-sectional view illustrating an example of a plurality of gas sensors arranged along the arrangement direction of the plurality of electrodes 11. As an example, the plurality of gas sensors includes a first gas sensor 31 (an example of the gas sensor 30), a second gas sensor 32, and a third gas sensor 33. The plurality of gas sensors is disposed downstream of the plurality of electrodes 11, respectively. The second gas sensor 32 is provided in a first region 42 in the gas chamber 41. The first region 42 is set downstream of the first electrode 11a and the second electrode 11b. The first region 42 is a region where the gas that has passed straight between the first electrode 11a and the second electrode 11b reaches. The first region 42 is a region that intersects the center line between the first electrode 11a and the second electrode 11b. The first region 42 is a region overlapping with a gap formed between the first electrode 11a and the second electrode 11b in viewing from above the gas measurement device 1 in the gas flow direction. The first gas sensor 31 and the third gas sensor 33 are disposed in a second region 43 adjacent to the first region 42. The second region 43 is set downstream of the first electrode 11a and the second electrode 11b, and is a region other than the first region 42 in the gas chamber 41. The first gas sensor 31 is disposed closer to the first electrode 11a than the second electrode 11b. The third gas sensor 33 is disposed closer to the second electrode 11b than the first electrode 11a.

The first gas sensor 31 disposed near the first electrode 11a mainly detects negative ions whose traveling direction is changed to a direction approaching the first electrode 11a. The third gas sensor 33 disposed near the second electrode 11b mainly detects positive ions whose traveling direction is changed to a direction approaching the second electrode 11b. The second gas sensor 32 disposed at a position facing between the first electrode 11a and the second electrode 11b mainly detects non-ionized gas molecules.

(B) of FIG. 3 is a cross-sectional view illustrating an example of a plurality of gas sensors arranged in the second region 43. A first gas sensor 31 (an example of the gas sensor 30) and a second gas sensor are disposed in the second region 43 adjacent to the first region 42 where the gas that has passed straight between the first electrode 11a and second electrode 11b reaches. The first gas sensor 31 mainly detects negative ions whose traveling direction is changed to a direction approaching the first electrode 11a. The second gas sensor 32 mainly detects negative ions whose traveling direction has been changed to a direction approaching the second electrode 11b. Since the traveling direction of the non-ionized gas molecules does not change in a direction approaching the first electrode 11a and the second electrode 11b, the non-ionized gas molecules are not detected by the first gas sensor 31 and the second gas sensor 32.

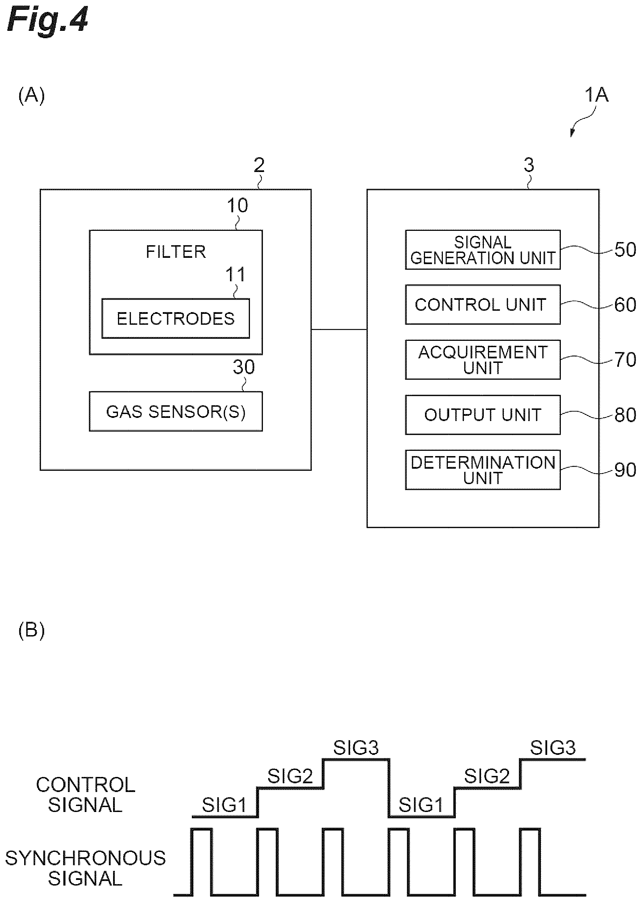

[Control Circuit of Gas Measurement Device] (A) of FIG. 4 is a block diagram illustrating an example of the gas measurement device 1A according to an embodiment. The gas measurement device 1A includes a measurement unit 2 (an example of a gas measurement device) and a circuit unit 3. The circuit unit 3 includes a signal generation unit 50, a control unit 60, an acquirement unit 70, an output unit 80, and a determination unit 90. The circuit unit 3 may be formed of, for example, an electric circuit. The circuit unit 3 may be configured by, for example, a general-purpose computer including an arithmetic device such as a central processing unit (CPU), a storage device such as a read only memory (ROM), a random access memory (RAM), and a hard disk drive (HDD), and a communication device.

The signal generation unit 50 outputs a control signal and a synchronous signal to the control unit 60 and the acquirement unit 70. The control signal is a signal that determines magnitude (which may be a potential difference) of the voltage or current applied to the electrode 11 by the power supply 12. The synchronous signal is a signal for determining the operation timing of the control unit 60 and the acquirement unit 70. Based on the control signal and the synchronous signal, the control unit 60 controls the power source 12 so as to apply voltage or current with the magnitude determined by the control signal to the electrode 11 at a timing determined by the synchronous signal. (B) of FIG. 4 is an example of the control signal and the synchronous signal. In (B) of FIG. 4, the control signal is a signal corresponding to the magnitude of three kinds of voltages SIG1, SIG2, and SIG3. The synchronous signal is a rectangular wave based on an oscillator such as a crystal oscillator.

The control unit 60 outputs a signal for controlling the power supply 12 according to the control signal when the rectangular wave of the synchronous signal is received a predetermined number of times. As an example, the control signal changing in three steps of (B) of FIG. 4 controls the magnitude of the voltage in three steps. The magnitude of the voltage may be steplessly controlled. In this case, the control signal indicates a triangular wave or a sine wave.

The acquirement unit 70 acquires a detection value detected by the gas sensor at a timing determined by the synchronous signal when the rectangular wave of the synchronous signal is received a predetermined number of times. Therefore, the acquirement unit 70 can acquire the detection value corresponding to the change in the magnitude of the voltage or current caused by the control signal. The predetermined number of times of receiving the rectangular wave when the acquirement unit 70 acquires the detection value may be equal to or greater than the number of times of receiving the rectangular wave when the control unit 60 outputs the control signal. By making the timing at which the acquirement unit 70 acquires the detection value later than the timing at which the control unit 60 outputs the control signal, the acquirement unit 70 can acquire the detection value when the number of gas molecules that have passed between the electrodes 11 becomes large after the magnitude of the voltage or current has changed.

The output unit 80 outputs the control signal and the detection value acquired from the acquirement unit 70 in association with each other.

The determination unit 90 determines a gas species based on the pre-acquired relationship, the detection value and the control signal output by the output unit 80. The pre-acquired relationship is a relationship between the gas species, the detection value, and the control signal. The combination of the gas species, the detection value, and the control signal is acquired in advance and stored as, for example, a gas characteristic table. The determination unit 90 determines the gas species with reference to the gas characteristic table based on the combination output by the output unit 80.

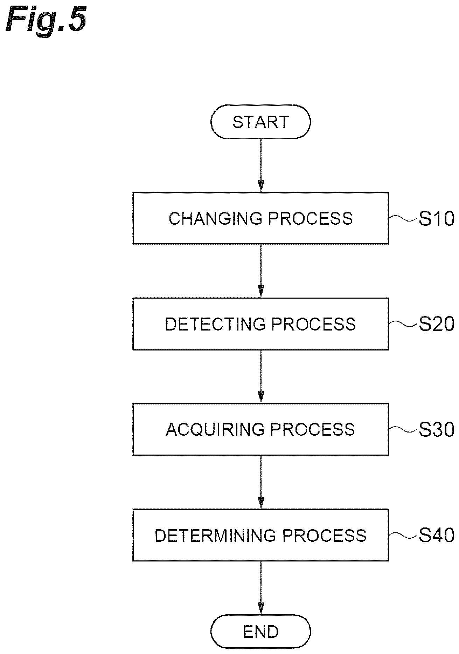

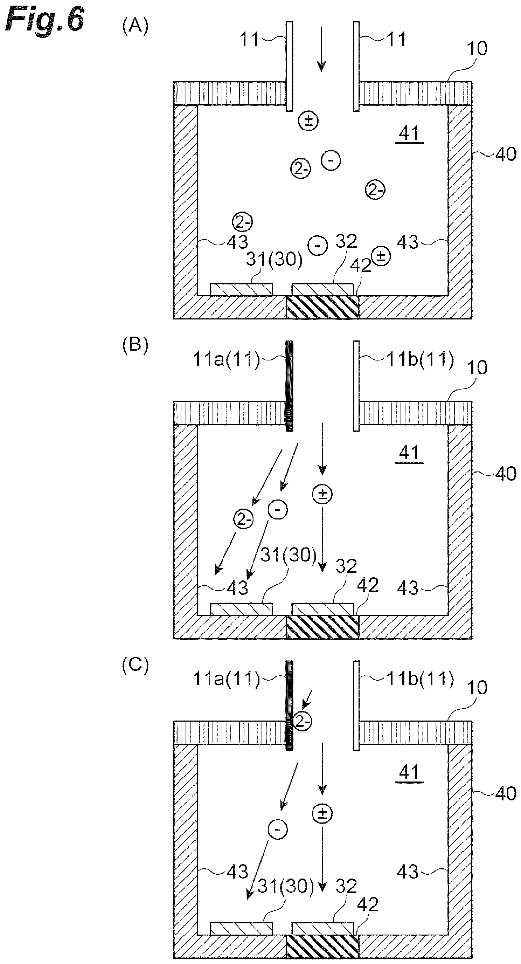

[Operation of Gas Measurement device] FIG. 5 is a flowchart illustrating an example of the gas measurement method according to the embodiment. The steps of the flow chart shown in FIG. 5 illustrate the operation of the gas measurement device 1A. FIG. 6 is a schematic view illustrating an example of a change in the traveling direction of the gas molecules due to the strength of the electrostatic force of the electrode 11. As an example, the gas to be detected is a mixed gas containing carbon monoxide (CO), chlorite ions (ClO⁻) and sulfate ions (SO₄²⁻). A first gas sensor 31 and A second gas sensor 32 are disposed downstream of the plurality of electrodes. The first gas sensor 31 is disposed in the second region 43 close to the first electrode 11a. The second gas sensor 32 is disposed in the first region 42 corresponding to between the first electrode 11a and the second electrode 11b.

As shown in FIG. 5, first, the control unit 60 changes the magnitude of the voltage or current applied to the electrodes 11 arranged apart from each other so as to have a gap through which gas can pass, based on the control signal and the synchronous signal that determines the timing (Step S10). (A) of FIG. 6 shows the traveling direction of gas molecules passing between the electrodes 11 when the control signal is SIG1. As an example, the control unit 60 does not apply voltage or current to the electrode 11 when the control signal is SIG1. Therefore, no electrostatic force is generated in the electrode 11. The traveling directions of carbon monoxide (CO), ionized chlorite ions (ClO⁻) and sulfate ions (SO₄²⁻) do not change.

(B) of FIG. 6 shows the traveling direction of gas molecules passing between the electrodes 11 when the control signal is SIG2. The control unit 60 applies voltage or current to the electrode 11 when the control signal is SIG2. The electrostatic force is generated between the first electrode 11a and the second electrode 11b to change the traveling direction of the negative ions to a direction approaching the first electrode 11a. Therefore, the traveling direction of the ionized chlorite ion (ClO⁻) and sulfate ion (SO₄²⁻) changes to a direction approaching the first electrode 11a when the control signal is SIG2. In this case, the first gas sensor 31 mainly detects the ionized chlorite ion (ClO⁻) and sulfate ion (SO₄²⁻) (Step S20). The second gas sensor 32 mainly detects carbon monoxide (CO) whose traveling direction does not change (Step S20).

(C) of FIG. 6 shows the traveling direction of the gas molecules passing between the electrodes 11 when the control signal is SIG3. The control unit 60 applies a larger voltage or current to the electrode 11 than in the case where the control signal is SIG2, when the control signal is SIG3. The stronger electrostatic force is generated between the first electrode 11a and the second electrode 11b than in the case where the control signal is SIG2. Therefore, the traveling direction of the divalent sulfate ion (SO₄²⁻) is largely changed to a direction approaching the first electrode 11a, and the divalent sulfate ion is adsorbed to the first electrode 11a, when the control signal is SIG3. In this case, the first gas sensor 31 mainly detects the ionized chlorite ion (ClO⁻) (Step S20).

Next, the acquirement unit 70 acquires the first measurement value output by the first gas sensor 31 and the second measurement value output by the second gas sensor 32 based on the synchronous signal (Step S30).

The determination unit 90 determines the gas species based on the gas characteristic table, the first measurement value, the second measurement value and the control signal (Step S40). The first measurement value, the second measurement value, and the control signal are acquired by the acquirement unit 70. From the pre-acquired relationship between the magnitude of the voltage or current indicated by the control signal and the measured value, it is determined that the gas to be detected contains carbon monoxide (CO), ionized chlorite ions (ClO⁻), and sulfate ions SO₄²⁻).

[Summary of Embodiment] According to the gas measurement devices 1, 1A, the plurality of electrodes 11 are provided, and electrostatic force is generated between the electrodes by applying voltage or current to the plurality of electrodes 11. The gas passes between the electrodes. At this time, the traveling direction of the ionized gas molecules contained in the gas changes due to the electrostatic force. The traveling direction of the non-ionized gas molecules does not change. Therefore, the gas measurement devices 1, 1A can change the type of gas molecules that can reach the gas sensor 30 by changing the magnitude of the electrostatic force. Therefore, the gas measurement devices 1, 1A can detect a plurality of types of gases with a simple configuration as compared to a gas measurement device including a plurality of types of reactive membranes.

The traveling direction of the ionized gas molecules changes to the direction of either the anode or the cathode of the plurality of electrodes 11. Therefore, the gas measurement devices 1, 1A can detect a plurality of types of gases whose traveling directions have changed.

The gas measurement device 1A can output the detection value and the synchronous signal in association with each other. The gas measurement device 1A and the gas measurement method can determine the gas species of a mixed gas containing gas molecules traveling in different directions.

[Modification] While various exemplary embodiments have been described above, various omissions, substitutions and changes may be made without being limited to the exemplary embodiments described above.

In a case where the first gas sensor 31 (an example of the gas sensor 30) and the second gas sensor 32 (an example of another gas sensor) are disposed downstream of the plurality of electrodes, the second gas sensor 32 may be configured to mainly detect gas molecules of a type different from the gas molecules detected by the first gas sensor 31. In this case, by providing a plurality of gas sensors having different gas detection performances downstream of the plurality of electrodes 11, gas measurement accuracy can be improved as compared with a gas measurement device including a gas sensor having a single type of gas detection performance downstream of the electrodes 11.

The filter 10 and the gas sensor 30 may be formed separately and then assembled together. The filter 10 and the gas sensor 30 may be integrally manufactured.

The signal generation unit 50 may be integrated with the control unit 60. The acquirement unit 70 may be integrated with the output unit 80. The output unit 80 may be integrated with the determination unit 90.

The gas measurement device 1 may be configured not to include the base member 40 and the gas chamber 41. In this case, the gas measurement device 1 is configured such that the electrode 11 of the filter 10 adheres to the gas sensor 30. The gas measurement device 1 may include M types of gas sensors (M is an integer of 2 or more). When M is 3 or more, the M types of gas sensors may include the same type of sensor. The intensity of the electrostatic force of the electrode 11 may be controlled in N stages (N is an integer of 2 or more). In this case, the gas measurement device 1 can measure the gas in a combination of M × N types at maximum.

The gas measurement device 1A may be configured not to include the determination unit 90. In this case, the output unit 80 of the gas measurement device 1A outputs the measured value and the control signal to the outside. The gas measurement device 1A may acquire the relationship between the measured value and the control signal in advance by simulation. In this case, the intensity of the electrostatic force of the electrode 11 and the electrostatic force generated between the ionized gas molecules are obtained by calculation. The relationship between the measured value and the control signal may be calibrated with a known gas. In this case, the relationship between the control signal and the strength of the electrostatic force of the electrode 11 and the output characteristic of the gas sensor 30 are calibrated based on the known gas in which the gas species and the valence of the ionized gas molecules are specified.

### Reference Signs List

1, 1A···gas measurement device, 2···measurement unit, 3···circuit unit, 10···filter, 11···electrode, 12···power source, 30···gas sensor, 40···base member, 41···gas chamber, 42 ···second region 43···second region, 50···signal generation unit, 60···control unit, 70··· acquirement unit, 80···output unit, 90···determination unit.

## Claims

1. A gas measurement device comprising:
a plurality of electrodes arranged apart from each other so as to have a gap through which gas can pass, each electrode connected to a power source for applying voltage or current; and
a gas sensor configured to detect gas molecules passing between the electrodes.

2. The gas measurement device according to claim 1, further comprising a plurality of gas sensors including the gas sensor,
wherein the plurality of gas sensors is arranged along an arrangement direction of the plurality of electrodes.

3. The gas measurement device according to claim 2,
wherein the plurality of gas sensors includes another gas sensor configured to detect gas molecules of a gas species different from the gas species of the gas molecules detected by the gas sensor.

4. The gas measurement device according to any one of claims 1 to 3,
wherein a first region where the gas passing straight between the electrodes reaches and a first region adjacent to a second region are set, and the gas sensor is disposed in the second region.

5. The gas measurement device according to any one of claims 1 to 4, further comprising:
a signal generation unit configured to output a synchronous signal for determining timing;
a control unit configured to control a power supply so that the voltage or current with magnitude determined by a control signal is output at the timing determined by the synchronous signal, based on the control signal for determining the magnitude of the voltage or current and the synchronous signal;
an acquirement unit configured to acquire a detection value of the gas sensor at a timing determined by the synchronous signal; and
an output unit configured to output the detection value and the control signal in association with each other.

6. The gas measurement device according to claim 5, further comprising a determination unit configured to determine a gas species based on a pre-acquired relationship, the detection value, and the control signal,
wherein the pre-acquired relationship is a relationship between the gas species, the detection value, and the control signal.

7. A gas measurement method comprising:
applying voltage or current with magnitude determined by a control signal at a timing determined by a synchronous signal to a plurality of electrodes spaced apart from one another so as to have a gap through which gas can pass, based on the control signal that determines the magnitude of the voltage or current and the synchronous signal that determines the timing;
detecting gas molecules passing between the electrodes by a gas sensor;
acquiring a detection value detected by the gas sensor at a timing determined by the synchronous signal; and
determining a gas species based on a pre-acquired relationship, the acquired detection value and the control signal, wherein the pre-acquired relationship is relationship between the gas species, the control signal, and the detection value.
